(19) Europäisches Patentamt
European Patent Office
Office européen des brevets

(11) Publication number: **0 193 415 B1**

(12) # EUROPEAN PATENT SPECIFICATION

(45) Date of publication of patent specification: **26.06.91**

(51) Int. Cl.[5]: **C07D 491/10**, C07D 495/10, C07D 513/10, A61K 31/41, //(C07D491/10,311:00,235:00)

(21) Application number: **86301530.1**

(22) Date of filing: **04.03.86**

The file contains technical information submitted after the application was filed and not included in this specification

(54) **Spiro-3-heteroazolidine compounds and salts thereof, their preparation and pharmaceutical agents thereof.**

(30) Priority: **04.03.85 JP 41234/85**

(43) Date of publication of application:
**03.09.86 Bulletin 86/36**

(45) Publication of the grant of the patent:
**26.06.91 Bulletin 91/26**

(84) Designated Contracting States:
**CH DE FR GB IT LI**

(56) References cited:
**EP-A- 0 028 485**
**EP-A- 0 092 386**
**EP-A- 0 129 747**
**GB-A- 2 028 318**
**GB-A- 2 080 304**
**US-A- 4 130 714**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Kurono, Masayasu**
**1-7-17, Kakeage Minami-ku**
**Nagoya-shi Aichi-ken(JP)**
Inventor: **Yamaguchi, Takuji**
**174-1, Ohaza-Higashikata**
**Kuwana-shi Mie-ken(JP)**
Inventor: **Usui, Toshinao**
**5-45, Minami-uzura**
**Gifu-shi Gifu-ken(JP)**
Inventor: **Fukushima, Masato**
**2000-33, Ohaza Kita-toyama**
**Komaki-shi Aichi-ken(JP)**
Inventor: **Mizuno, Kuniharu**
**53, Ohaza-oguchi Aza-baba Ohguchi-cho**
**Niwa-gun Aichi-ken(JP)**
Inventor: **Matsubara, Akira**
**126, Kitayama Kitayama-cho**
**Owari-asahi-shi Aichi-ken(JP)**

(74) Representative: **Diamond, Bryan Clive et al**
**Gee & Co., Chancery House, Chancery Lane**
**London WC2A 1QU(GB)**

**EP 0 193 415 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel spiro-3-heteroazolidine compounds and salts thereof, processes for the preparation of these compounds as well as pharmaceutical agents comprising at least one of the compounds to cure or prevent complications caused by diabetes.

Hithereto, various studies have been made to find an effective agent for curing diabetes, and which can be orally dosed. As a result, various agents therefor, each of which comprises as an effective component sulfonyl urea, mesooxalate or a guanidine derivative or the like have been developed and marketed, but these merely treat the symptoms of the hyperglycoplasmia due to the diabetes. It has been known there may be caused by diabetes specific chronic complications such as diabetic cataract, diabetic neuropathy and diabetic retinopathy but there is almost no effective agent for curing such complications and thus no effective therapeutic system has been available.

Therefore, hithereto, studies have also been made to develop an effective agent for curing such intractable diseases due to diabetes, but hitherto without significant success. As one of these studies, a search has been made for inhibitors of aldose reductase enzymes, since the enzyme reduces, in vivo in human and other animals, aldoses such as glucose and galactose into corresponding polyols such as sorbitol and galactitol and it has been known that said complications appear when the formed sorbitol and galactitol are accumulated at the crystalline lens, peripheral nerve, kidney or the like in patients having diabetes or galactosemia ["Jap. J. Opthalmol." Vol. 20, page 399 (1976); "Int. Congr. Ser. Excerpta Med." Vol. 403, page 594 (1977); and "Metabolism" Vol. 28, page 456 (1979)].

GB-A-2080-304 discloses hydantoin derivatives, and salts thereof, of the type shown by formula I-A herein below but wherein the benzene ring may carry, in place of Y herein, at any of the four free positions, one or two substituents selected from halogen, lower alkyl and lower alkoxy, and V and W are hydrogen (not both of them), lower alkyl or phenyl.

The present compounds differ therefrom by the V and W substituents (not alkyl or alkoxy).

EP-A-0092-386 discloses hydantoin derivatives, and salts thereof, also as shown in the present formula I-A but without any V or W substituent and with a $CH_3S$- or $CH_3S(O)$- substituent on that ring.

Both these prior types of compounds are useful in the treatment of diabetes mellitus. The present compounds have superior effects thereto on the liver metabolism.

An object of the invention is to provide a novel inhibitor of aldose reductase enzymes to prevent an accumulation of sorbitol and galactitol in vivo and in turn permit the prevention and curing of complications due to diabetes.

According to the invention, we provide novel spiro-3-heteroazolidine compounds represented by the general formula

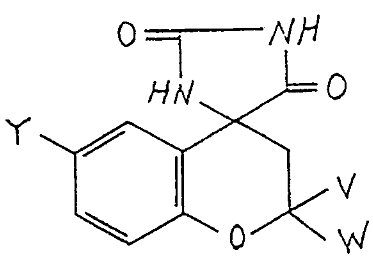

or

(I-A)

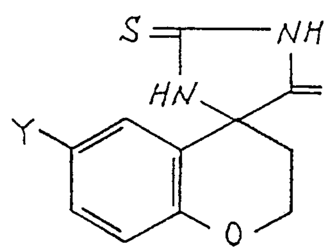

(I-B)

wherein one of V and W represents a hydrogen atom, the other of V and W represents a hydroxymethyl radical, aminomethyl radical, halomethyl radical, -COOR, or

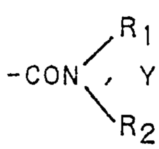

, Y represents a hydrogen atom or halogen atom, R is a hydrogen atom, alkyl group having 1 to 6 carbon atoms or $-(CH_2CH_2O)4)CH_3$, $R_1$ and $R_2$ are the same or different and each represents a hydrogen atom, alkyl radical having 1 to 6 carbon atoms, $-(CH_2CH_2O)_4CH_3$, or methoxy substituted phenyl radical, and pharmaceutically acceptable salts thereof.

It has been confirmed that the compounds of the invention have an effective inhibition action upon aldose reductase enzymes and that their toxity is quite low.

In these compounds, each alkyl group for R may be a straight-chain or branched-chain alkyl radical. Examples of straight-chain alkyl radicals are those having 1 to 6 carbon atoms, for instance methyl, ethyl, n-propyl, n-butyl, n-pentyl or n-hexyl; and of branched-chain alkyl radicals there are isopropyl, isobutyl, s-butyl and t-butyl.

A halogen atom for Y can be any of fluorine, chlorine, bromine and iodine, but fluorine is preferred.

A halomethyl radical for V or W can be fluoromethyl, chloromethyl, bromomethyl or iodomethyl.

A salt of a compound (I) must be acceptable for use as an effective component in pharmaceutical agents; examples thereof are those with cations such as sodium, potassium, calcium and magnesium.

Each of the compounds (I) according to the invention have two asymmetric carbon atoms in its structure and thus have two kinds of stereo isomers and optical isomers thereof. All these isomers are included in the scope of the invention.

According to one process of the invention, the class of compounds of the formula (I-A) can be prepared by reacting a compound represented by the formula

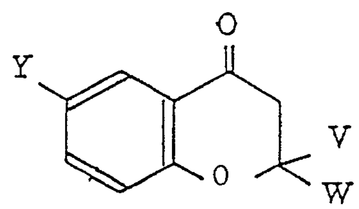

(II)

wherein V, W, and Y are as defined above, with a metallic cyanide and ammonium carbonate.

Sodium cyanide or potassium cyanide may be used as the metallic cyanide. The reaction may be carried out in the presence of a solvent and at 50 to 150 °C for about 4 hours to 2 days. Suitable solvents are water, lower alkanols (e.g. methanol, ethanol or propanol), lower alkanoamides (e.g. acetylamide), cyclic ethers (e.g. dioxane or tetrahydrofuran), lower alkylene glycols (e.g. ethylene glycol or triethylene glycol), and N,N-dialkylamides (e.g. N,N-dimethylformamide or N,N-diethylformamide). It is preferred to mix the compound (II), metallic cyanide and ammonium carbonate in a mol ratio of 1.0 : 1.2 : 2.5 to 1 : 3 : 8. After completion of the reaction, an aqueous reaction solution (when the solvent is water and if the solvent is not water, the reaction solution diluted with water) is acidified to cause a precipitation of the desired compound whereby isolation thereof can easily be carried out.

It can be deduced that the product (I-A) of this process will comprise two kinds of isomers based on the presence of two asymmetric carbon atoms, but according to the process of the invention, one of the isomers is predominantly and stereoselectively formed, which can easily be isolated by a mere crystallization and shows excellent pharmaceutical activities including an inhibition of galactitol accumulation.

When any of the compounds (II) used as the starting material for the process according to the invention is not commercially available, it can be synthesized as follows. For instance, a compound of the formula (II), wherein the substituents V = H and W = COOH, namely the compound represented by the formula

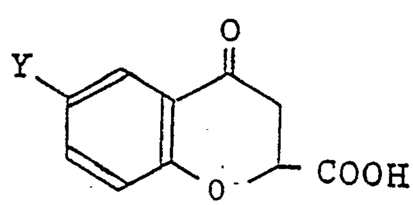

(II-a)

wherein Y is as defined above, can be prepared by starting from chromanones ["Ann. Chim." (Rome), 57-(10), pages 1045 to 1072 and 58(10), pages 1155 to 1162 (1968)] and in accordance with the following route:

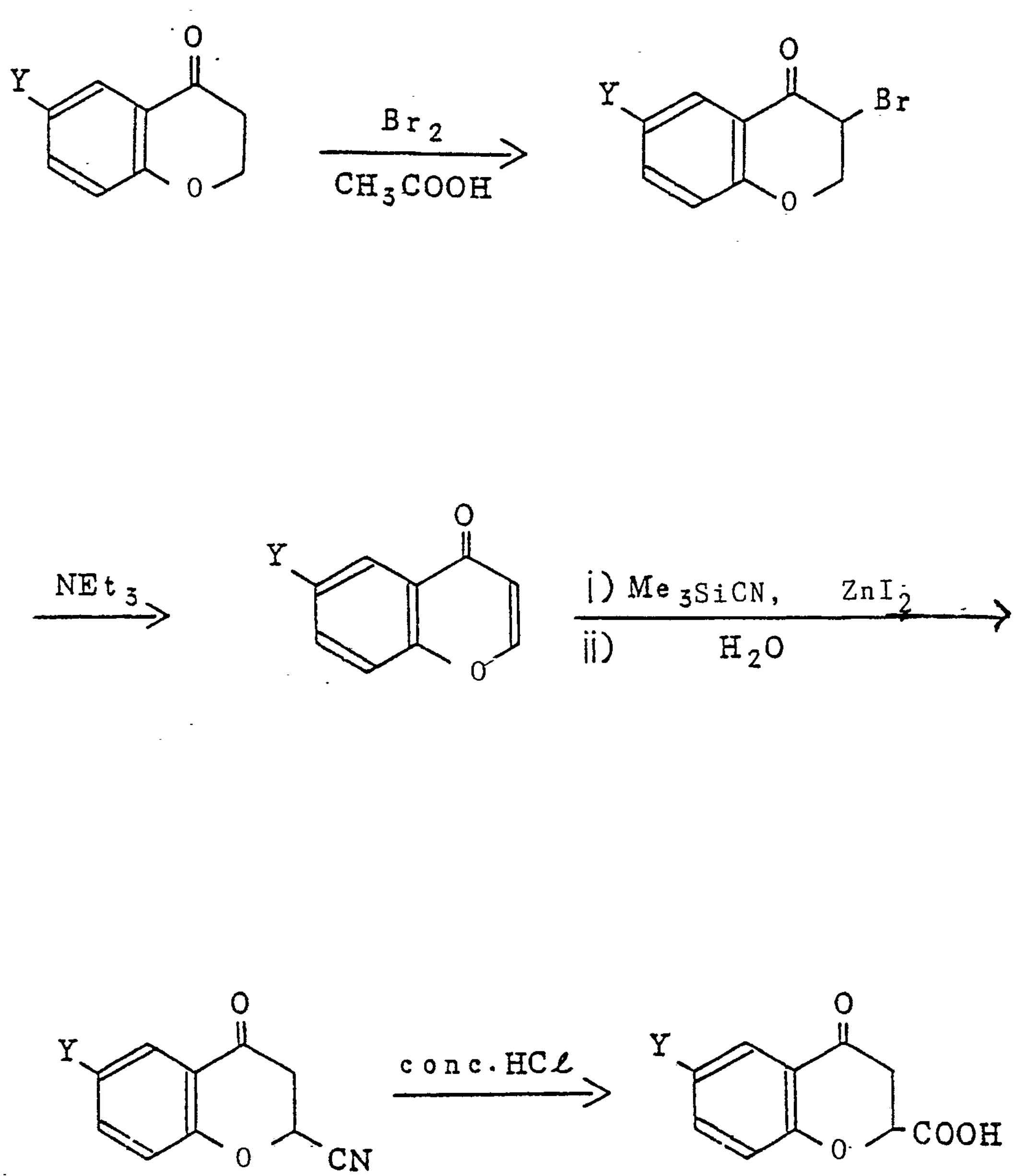


wherein X, Y and Z are as defined above.

On the other hand, another class of compound among those of the formula (II), can be prepared in accordance with a process as disclosed by H.J Kabbe in "Synthesis", December 1978 , page 886, and an ester or amide derivative can be synthesized by reacting the compound II-a or II-b with an alcohol or amine.

In synthesizing the ester or carboxamide derivative among the compounds I-A, it is found that the yield becomes lower when the corresponding compound of the formula II is directly hydantoinized. In this case, therefore, it is convenient that in the first place, the compound shown by the formula II-a or II-b is hydantoinized to synthesize the corresponding 2-carboxylic acid, and then the acid is treated to be converted into the desired compound. Thus, sulfuric acid, hydrochloric acid or the like mineral acid is reacted on the carboxylic acid in methanol, ethanol, propanol or a like alkanol, the carboxylic acid and and alcohol compound are condensed in benzene, toluene or a like non-polar solvent and with use of an aromatic sulfonic acid; or the carboxylic acid and an alcohol compound are condensed with dicyclohexylcarbodiimide or the like condensation agent to form the ester derivative, or the carboxylic acid and an amine are condensed in pyridine, dioxane or a like inert solvent and with use of tetrachlorosilane, dicyclohexylcarbodiimide or the like condensation agent to form the carboxamide derivative.

The hydroxymethyl, halomethyl and aminomethyl derivatives for V or W can also be prepared by starting from said carboxylic acid. The following are routes for synthesizing these derivatives.

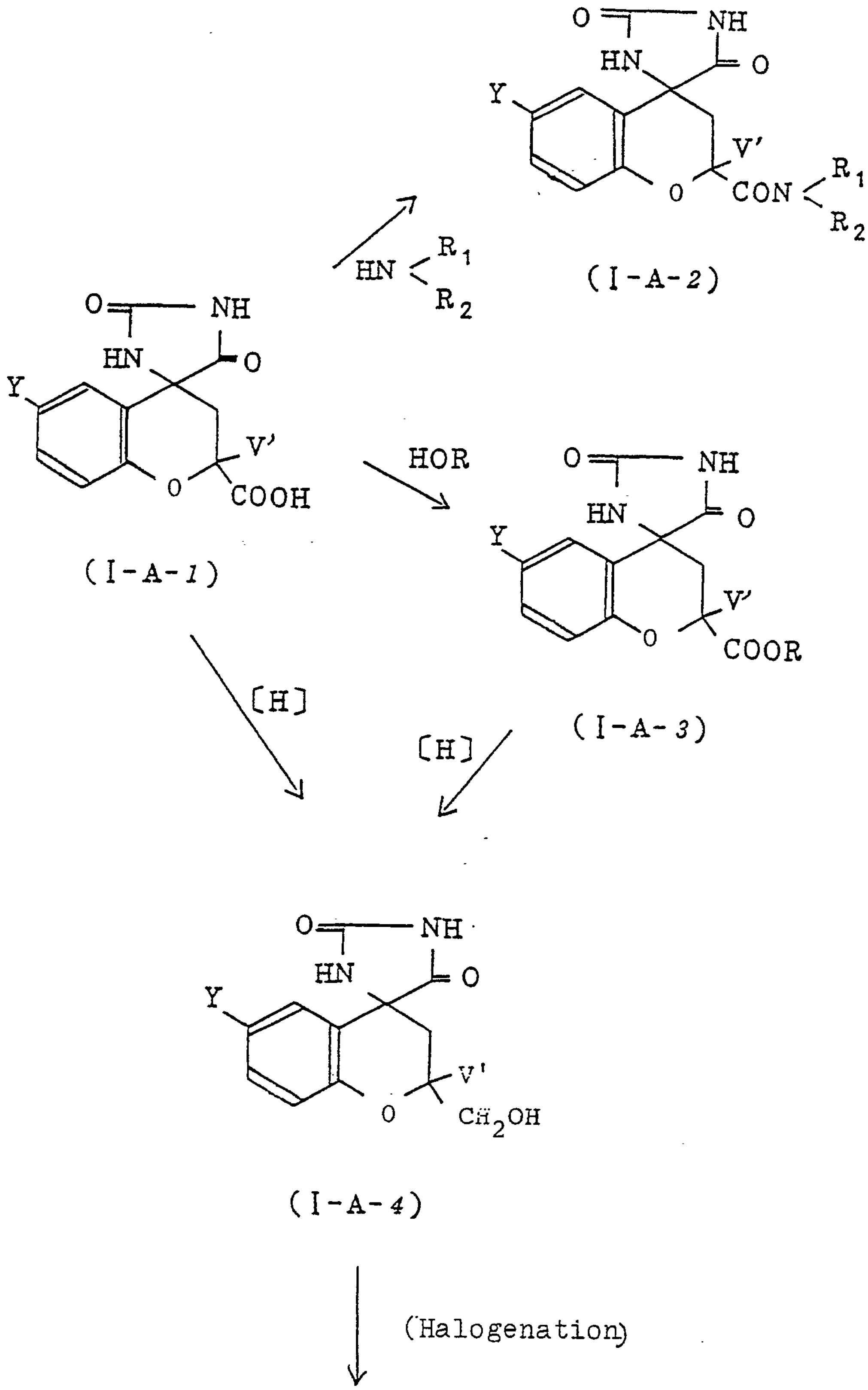


(I-A-1)

(I-A-2)

(I-A-3)

(I-A-4)

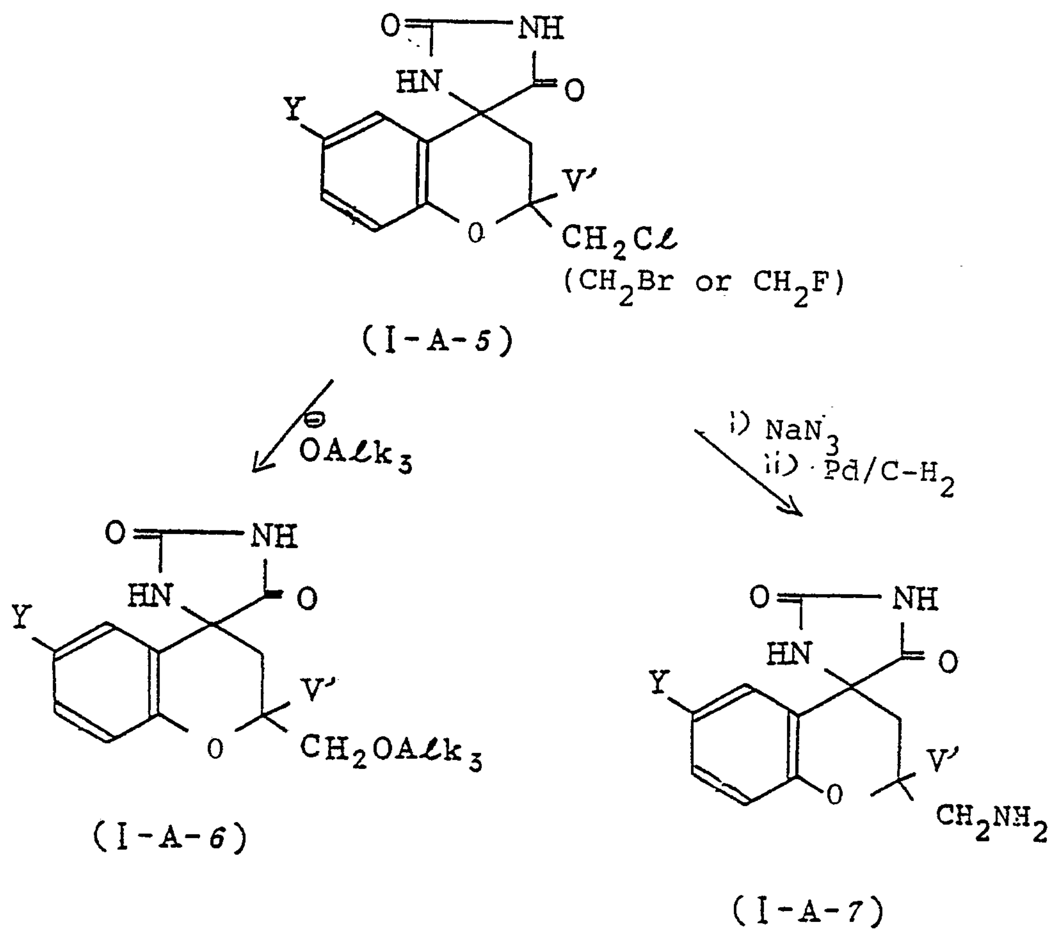


(I-A-5)

(I-A-6)

(I-A-7)

wherein R, $R_1$, $R_2$, and Y are as defined above, V' is a hydrogen atom and Alk means an alkyl group.

A compound among these of the formula (I-B)

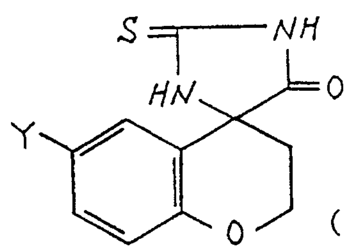
(I-B)

wherein V, W and Y have the meanings given above can be synthesized by treating with potassium thiocyanate the compound III:

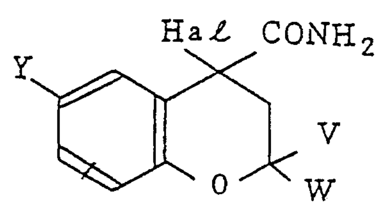
(III)

This reaction is carried out at 60 to 150°C with use of acetone, acetic acid, a cyclic ether, N,N-dialkylamide or a like solvent; it is preferable to use acetic acid as solvent, at its reflux temperature.

The compound III used as starting material for this process can be prepared by a known method, for instance by halogenizing by use of thionylchloride, phosphorous tribromide or the like a compound represented by the formula

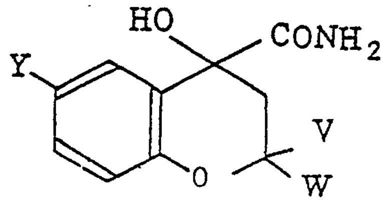

(IV)

wherein V, W, Y and have the meanings as defined above.

The spiro-3-heteroazolidine compounds according to the invention show a quite low toxicity of higher than 6000 mg/kg in oral dosage, and a high anti- or inhibition activity to aldose reductase enzymes. Especially, 6-flouro-2,3-dihydro-2',5'-dioxo-spiro (4H-1-benzo-pyran-4,4'-imidazolidine)-2-carboxamide and 6-fluoro-2,3-dihydro-N,N-dimethyl-2',5'-dioxo-spiro (4H-1-benzopyran-4,4'-imidazolidine)-2-carboxamide are excellent in inhibition of polyol accumulation in the sciatic nerve. This indicates that these compounds exert a strong action on the nervous system and thus the compounds are believed to be effective in curing of peripheral neuropathy, which has been considered as an incurable disease among the complications due to diabetes. The low toxicity of the compounds according to the invention allows a continuous dosage thereof and this constitutes an important factor for curing chronic complications due to diabetes.

The invention will now be further illustrated by reference to the following Examples of preparing the compounds and pharmaceutical preparations according to the invention, as well as Examples showing the pharmaceutical properties and effects of the compounds.

Reference Example 1

a) 3-Bromo-6-fluorochromanone

To a solution of 6-fluorochromanone 99.6g, 0.6 mol) in 500 ml of acetic acid, 96.0g (0.6 mol) of bromine were added dropwise at a rate so as to maintain a reaction temperature of 25° C. After stirring the reactants for 2.0 hours at 25° C, the reaction mixture was poured into 1.2 litre of cracked ice with stirring. The resultant precipitate was subsequently filtered, washed with water and then dried in air to give 140g (95.2%) of the desired compound.

Melting point: 56 - 57℃

IR spectrum ($\nu_{max}^{KBr}$) $cm^{-1}$: 1690, 1620

NMR spectrum ($CDCl_3$) $\delta$ ppm:

4.58 (3H, s)

6.85 - 7.68 (3H, m)

Mass spectrum (EI/DI) m/z: 244 ($M^+$), 165, 148

b) 6-Fluoro-4H-1-benzopyran-4-one

A solution of 3-bromo-6-fluorochromanone (140g, 0.57 mol)(obtained by the process described above part a) in 1.5 litre of triethylamine was heated at reflux temperature for 1.5 hours. After cooling the reaction mixture, the resulting precipitate was filtered and the filtrate was evaporated in vacuo to combine the residue with solids obtained through said filtration. The combined solids were partitioned between 1.2 litre of dichloromethane and 1.2 litre of 2N-hydrochloric acid. An organic layer was washed with water, dried over

anhydrous sodium sulfate,filtered and evaporated in vacuo to dryness. The resultant residue was recrystallized from ethyl acetate to give 72.2g (77.2%) of the desired compound.

Melting point: 165 - 168℃

IR spectrum ($\nu_{max}^{KBr}$) $cm^{-1}$: 1660, 1640, 1620

NMR spectrum ($CDCl_3$) δ ppm:

6.33 (1H, d, J=6Hz)

7.17 - 7.97 (3H, m)

7.82 (1H, d, J=6Hz)

Mass spectrum (EI/DI) m/z: 164 ($M^+$), 136

c) 6-Fluoro-3,4-dihydro-4-oxo-2H-1-benzopyran-2-carbonitrile

To a mixture of 72.2g (0.44 mol) of 6-fluoro-4H-1-benzopyran-4-one obtained by the process as described in part b and 1.39g (4.4 mmol) of zinc iodide in 610 ml of dry ether, 101g (1.0 mol) of trimethylsilylcyanide were added under stirring. The mixture was heated at reflux temperature for 24 hours. After cooling the reaction mixture, the solution was poured into 500 ml of methanol, stirred for one hour at room temperature and evaporated in vacuo to dryness. Resulting residue was chromatographed on silica gel, eluting with dichloromethane to give 79.7g (94.9%) of the desired compound.

Melting point: 87 - 89℃

IR spectrum ($\nu_{max}^{KBr}$) $cm^{-1}$: 1690, 1617

NMR spectrum ($CDCl_3$) δ ppm:

3.12 (2H, d, J=6Hz)

5.43 (1H, t, J=6Hz)

6.83 - 7.73 (3H, m)

Mass spectrum (EI/DI) m/z: 191 ($M^+$), 164, 138, 110

d) 6-Fluoro-3,4-dihydro-4-oxo-2H-1-benzopyran-2-carboxylic acid

A solution of 6-fluoro-3,4-dihydro-4-oxo-2H-1-benzopyran-2-carbonitrile (78.3g, 0.41 mol)(obtained by the process as described in part c) in 760 ml of concentrated hydrochloric acid was heated at reflux temperature for 50 minutes. After cooling the reaction solution, 700 ml of water and 1 litre of ethyl acetate were added to carry out an extraction. Resulting organic layer was evaporated in vacuo to dryness. To the residue, 700 ml of saturated sodium biscarbonate was added and stirred for 30 minutes. The aqueous basic solution was washed with 500 ml of ethyl acetate, acidified with 6N-hydrochloric acid and extracted with 1 litre of ethyl acetate. The organic layer was washed with water, dried over anhydrous sodium sulfate, filtered and evaporated in vacuo to give 76.9g (89.3%) of the desired compound.

Melting point: 163 - 164℃

IR spectrum ($\nu_{max}^{KBr}$) $cm^{-1}$: 1750, 1660

NMR spectrum ($CDCl_3$) δ ppm:

3.07 (2H, d, J=6Hz)

5.10 (1H, t, J=6Hz)

7.11 - 7.60 (3H, m)

11.33 (1H, broad s)

Mass spectrum (EI/DI) m/z: 210 ($M^+$), 165, 138

Example 1

6-Fluoro-2,3-dihydro-2',5'-dioxo-spiro[4H-1-benzopyran-4,4'-imidazolidine]-2-carboxylic acid

A mixture consisting of potassium cyanide (37.1g, 0.57 mol), ammonium carbonate (164g, 1.7 mol) and 6-fluoro-3,4-dihydro-4-oxo-2H-1-benzopyran-2-carboxylic acid (60.0g, 0.29 mol)(obtained through the process as described in part d of the Reference Example 1) was heated at 65 - 70 °C for 24 hours under stirring and then at reflux temperature for 15 minutes. The reaction mixture was cooled to room temperature and then acidified to pH I with concentrated hydrochloric acid. Resulting precipitate was subsequently filtered, washed with water and then recrystallized from water to give 48.8g (60.1%) of the desired compound.

Melting point: 294 - 298℃ (dec.)

IR spectrum ($\nu_{max}^{KBr}$) $cm^{-1}$: 1770, 1750, 1720

NMR spectrum (DMSO-d ) δ ppm:

1.88 - 2.80 (2H, m)

5.23 (1H, dd)

6.83 - 7.38 (3H, m)

8.37 (1H, broad s)

11.07 (1H, broad s)

Mass spectrum (EI/DI) m/z: 280 ($M^+$), 262, 234, 219

Elementary analysis: $C_{12}H_9FN_2O_5$

Cal. : C, 51.43; H, 3.24; N, 10.00

Found: C, 51.15; H, 3.28; N, 9.98

The compound obtained through said process in this Example was a single diastereoisomer. From the mother liquor of recrystallization, another diastereisomer was obtained.

In evaluation of these isomers for ability to reduce or inhibit polyol increase in sciatic nerve of galactosemic rats, the potency of the major diastereoisomer was greater than that of the minor diastereoisomer.

Example 2

6-Fluoro-2,3-dihydro-2',5'-dioxo-spiro[4H-1-benzopyran-4,4'-imidazolidine]-2-carboxamide

To a solution of 6-fluoro-2,3-dihydro-2',5'-dioxo-spiro[4H-1-benzopyran-4,4'-imidazolidine]-2-carboxylic acid (2.8g, 0.01 mol) (obtained through the process as described in Example 1) in 30 ml of anhydrous pyridine, 1.2g (0.006 mol) of silicon tetrachloride were added at a temperature below 10 °C. After stirring for 15 minutes at room temperature, an excess amount of dry ammonia gas was perfused at temperature below 10 °C. The mixture was stirred for 18 hours at room temperature and poured into 100 ml of ethanol. Undissolved matter was filtered off and the filtrate was evaporated to dryness. To the remaining residue, 20 ml of water was added to stir for 30 minutes at room temperature. A forming precipitate was filtered and subsequently recrystallized from ethanol to give 2.0g (70.6%) of the desired compound.

Melting point: 286 - 300°C (dec.)

IR spectrum ($\nu_{max}^{KBr}$) $cm^{-1}$: 1770, 1720, 1670

NMR spectrum (DMSO-d ) δ ppm:

1.83 - 2.67 (2H, m)
5.17 (1H, dd)
6.93 - 7.33 (3H, m)
7.57, 7.80 (2H, broad s)
8.47 (1H, broad s)
11.07 (1H, broad s)

Mass spectrum (EI/DI) m/z: 279 ($M^+$), 262, 235, 219

Elementary analysis: $C_{12}H_{10}FN_3O_4$

Cal. : C, 51.62; H, 3.61; N, 15.05
Found: C, 51.79; H, 3.58; N, 14.98

Example 3

6-Fluoro-2,3-dihydro-N-methyl-2',5'-dioxo-spiro[4H-1-benzopyran-4,4'-imidazolidine]-2-carboxamide

To a solution of monomethylamine hydrochloride (1.6g, 0.024 mol) and 6-fluoro-2,3-dihydro-2',5'-dioxo-spiro[4H-1-benzopyran-4,4'-imidazolidine)-2-carboxylic acid (5.6g, 0.020 mol)(obtained through the process described in Example 1) in 60 ml of anhydrous pyridine, 2.4g (0.024 mol) triethylamine were added at temperature below 0 °C, followed by addition of 2.3g (0.013 mol) of silicon tetrachloride at the same temperature. After stirring the mixture for 18 hours at room temperature, the reaction solution was poured into ethanol. Undissolved matter therein was filtered off and the filtrate was evaporated to dryness. The remaining residue was chromatographed on silica gel, eluting with ethanol to give colorless crystals which were recrystallized from methanol to give 4.2g (71.2%) of refined desired compound.

Melting point: 297 - 300℃ (dec.)

IR spectrum ($\nu_{max}^{KBr}$) $cm^{-1}$: 1775, 1720, 1650

NMR spectrum (DMSO-$d_6$) δ ppm:

2.00 - 2.60 (2H, m)
2.68 (3H, d, J=5.0Hz)
5.07 (1H, dd)
6.67 - 7.37 (3H, m)
8.00 - 8.50 (1H, broad s)
8.33 (1H, broad s)
10.07 (1H, broad s)

Mass spectrum (EI/DI) m/z: 293 ($M^+$), 235, 192, 164

Elementary amalysis: $C_{13}H_{12}FN_3O_4$

Cal. : C, 53.24; H, 4.12; N, 14.33
Found: C, 53.14; H, 3.97; N, 14.16

Example 4

6-Fluoro-N-ethyl-2,3-dihydro-2',5,-dioxo-spiro[4H-1-benzopyran-4,4'-imidazolidine]-2-carboxamide

The procedure described in Example 3 was repeated except that ethylamine hydrochloride was employed as the starting material in the same molar amount (2.72g, 0.024 mol) in place of monomethylamine hydrochloride. In this particular case, 4.80g (78.1%) of the desired compound were obtained.

Melting point: above 300℃

IR spectrum ($\nu_{max}^{KBr}$) $cm^{-1}$: 1780, 1720, 1640

NMR spectrum (trifluoroacetic acid-$d_1$) δ ppm:

1.36 (3H, t, J=7.0Hz)
2.33 - 3.20 (2H, m)
3.62 (2H, q, J=7.0Hz)
5.62 (1H, dd)
7.00 - 7.30 (3H, m)

Mass spectrum (EI/DI) m/z: 307 ($M^+$), 235, 192

Elementary analysis: $C_{14}H_{14}FN_3O_4$

Cal. : C, 54.72; H, 4.59; N, 13.67
Found: C, 54.54; H, 4.55; N, 13.69

Example 5

6-Fluoro-2,3-dihydro-N,N-dimethyl-2',5'-dioxo-spiro[4H-1-benzopyran-4,4'-imidazolidine]-2-carboxamide

The procedure described in Example 3 was repeated except that dimethylamine hydrochloride was employed as the starting material in the same molar amount (1.96g, 0.024 mol) in place of monomethylamine hydrochloride. In this particular case, 4.60g (75.4%) of the desired compound were obtained.

Melting point: 285 - 293℃ (dec.)

IR spectrum ($\nu_{max}^{KBr}$) $cm^{-1}$: 1780, 1730, 1640

NMR spectrum (DMSO-$d_6$) $\delta$ ppm:

2.07 - 2.67 (2H, m)
2.90 (3H, s)
3.07 (3H, s)
5.57 (1H, dd)
6.77 - 7.20 (3H, m)
8.33 (1H, broad s)
10.03 (1H, broad s)

Mass spectrum (EI/DI) m/z: 307 ($M^+$), 262, 246, 235

Elementary analysis: $C_{14}H_{14}FN_3O_4$

Cal. : C, 54.72; H, 4.59; N, 13.68
Found: C, 54.73; H, 4.53; N, 13.53

Example 6

6-Fluoro-2,3-dihydro-N-propyl-2',5'-dioxo-spiro[4H-1-benzopyran-4,4'-imidazolidine]-2-carboxamide

To a solution of n-propylamine (1.4g, 0.024 mol) and 6-fluoro-2,3-dihydro-2',5'-dioxo-spiro[4H-1-benzopyran-4,4'-imidazolidine)-2-carboxylic acid (5.6g, 0.02 mol)(obtained through the process as described in Example 1) in 56.0 ml of anhydrous pyridine, 2.3g (0.013 mol) of silicon tetrachloride were added at a temperature below 20°C. After stirring for 15 hours at room temperature, the reaction mixture was poured into ethanol. Undissolved matter was filtered off and the filtrate was evaporated in vacuo to dryness. The remaining residue was chromatographed on silica gel, eluting with ethanol to give colorless crystals which were recrystallized from methanol to give 5.0g (77.5%) of the desired refined compound.

Melting point: 282 - 284°C

IR spectrum ($\nu_{max}^{KBr}$) $cm^{-1}$: 1780, 1730, 1680

NMR spectrum (trifluoroacetic acid-$d_1$) δ ppm:

1.07 (3H, t, J=7.0Hz)

1.30 - 2.00 (2H, m)

2.33 - 3.33 (2H, m)

3.53 (2H, broad t, J=7.0Hz)

5.58 (1H, dd)

6.96 - 7.33 (3H, m)

Mass spectrum (EI/DI) m/z: 321 ($M^+$), 235, 192

Elementary analysis: $C_{15}H_{16}FN_3O_4$

Cal. : C, 56.07; H, 5.02; N, 13.08

Found: C, 55.77; H, 5.06; N, 13.12

Example 7

6-Fluoro-2,3-dihydro-N-butyl-2',5'-dioxo-spiro[4H-1-benzopyran-4,4'-imidazolidine]-2-carboxamide

The procedure described in Example 6 was repeated except that n-butylamine was employed as the starting material in the same molar amount (1.8g, 0.024 mol) in place of n-propylamine. In this particular case, 4.40g (65.7%) of the desired compound were obtained.

Melting point: 286 -288℃

IR spectrum ($\nu_{max}^{KBr}$) $cm^{-1}$: 1775, 1720, 1630

NMR spectrum (trifluoroacetic acid-$d_1$) δ ppm:

1.03 (3H, broad t, J=7.0Hz)

1.23 - 2.10 (4H, m)

2.33 - 3.33 (2H, m)

3.56 (2H, broad t, J=7.0Hz)

5.60 (1H, dd)

6.76 - 7.30 (3H, m)

Mass spectrum (EI/DI) m/z: 335 ($M^+$), 235, 192

Elementary analysis: $C_{16}H_{18}FN_3O_4$

Cal. : C, 57.31; H, 5.41; N, 12.54

Found: C, 57.10; H, 5.47; N, 12.57

Example 8

6-Fluoro-2,3-dihydro-N-(4-methoxyphenyl)-2',5'-dioxo-spiro[4H-1-benzopyran-4,4'-imidazolidine]-2-carboxamide

a) The procedure described in Example 6 was repeated except that 4-methoxyphenylamine was employed as the starting material in the same molar amount (3.0g, 0.024 mol) in place of n-propylamine. In this particular case, 5.30g (68.4%) of the desired compound were obtained.

b) To a solution of 6-fluoro-2,3-dihydro-2',5'-dioxo-spiro[4H-1-benzopyran-4,4'-imidazolidine]-2-carboxylic acid (140mg, 0.5 mmol) (obtained through the process as described in said Example 1) and 4-methoxyphenylamine (63mg, 0.5 mmol) in 1.0 ml of N,N-dimethylformamide, a solution of dicyclohexylcarbodiimide (103mg, 0.5 mmol) in 1.0 ml of N,N-dimethylformamide was added at 5 °C. After stirring for 15 hours at room temperature, undissolved matter was filtered off. To the filtrate, 5 ml of 2N-hydrochloric acid were added and the mixture was stirred for 30 minutes. The formed precipitate was filtered and subsequently chromatographed on silica gel, eluting with ethyl acetate to give 78.3mg (40.7%) of the desired refined compound.

c) A mixture of 6-fluoro-3,4-dihydro-N-(4-methoxyphenyl)-4-oxo-2H-1-benzopyran-2-carboxamide (220mg, 0.7 mmol), potassium cyanide (97.5mg, 1.5 mmol) and ammonium carbonate (400mg, 4.2 mmol) in 2.1 ml of 60% aqueous ethanol was heated at 65 to 70 °C in a sealed tube for 40 hours. The reaction mixture was diluted with 5.0 ml of water and then acidified with 6N-hydrochloric acid to pH I. The formed precipitate was filtered and recrystallized from ethanol to give 67.3mg (25.0%) of the desired compound.

Melting point: 301 - 304℃ (dec.)

IR spectrum ($\nu_{max}^{KBr}$) $cm^{-1}$: 3300, 1775, 1730, 1640

NMR spectrum (DMSO-$d_6$) δ ppm:

2.00 - 2.73 (2H, m)
3.73 (3H, s)
5.33 (1H, dd)
6.80 - 7.80 (7H, m)
8.40 (1H, s)
10.13 (1H, s)
11.07 (1H, broad s)

Mass spectrum (EI/DI) m/z: 385 ($M^+$), 236

Elementary analysis: $C_{19}H_{16}FN_3O_5$

Cal. : C, 59.22; H, 4.19; N, 10.91
Found: C, 59.01; H, 4.12; N, 10.96

Example 9

6-Fluoro-2,3-dihydro-N-(3,6,9,12-tetraoxatridecyl)-2',5'-dioxo-spiro[4H-1-benzopyran-4,4'-imidazolidine]-2-carboxamide

The procedure described in Example 6 was repeated except that 3,6,9,12-tetraoxatridecylamine was employed as the starting material in the same molar amount (5.0g, 0.024 mol) in place of n-propylamine. In this particular case, 7.20g (77.1%) of the desired compound were obtained.

Melting point: 162 - 164℃

IR spectrum ($\nu_{max}^{KBr}$) $cm^{-1}$: 1770, 1720, 1640

NMR spectrum (DMSO-$d_6$) δ ppm:

1.90 - 2.80 (2H, m)
3.33 (3H, s)
3.30 - 3.80 (16H, m)
5.23 (1H, dd)
6.90 - 7.30 (3H, m)
8.25 (1H, broad s)
8.45 (1H, broad s)
11.01 (1H, broad s)

Mass spectrum (EI/DI) m/z: 469 ($M^+$), 305

Elementary analysis: $C_{21}H_{28}FN_3O_9$

Cal. : C, 53.73; H, 6.01; N, 8.95
Found: C, 53.85; H, 6.19; N, 8.98

Example 10

6-Fluoro-2,3-dihydro-2',5'-dioxo-spiro[4H-1-benzopyran-4,4'-imidazolidine]-2-carboxylic acid methyl ester

To a solution of 6-fluoro-2,3-dihydro-2',5'-dioxo-spiro[4H-1-benzopyran-4,4'-imidazolidine]-2-carboxylic acid (2.8g, 0.01 mol) (obtained through the process as described in Example 1) in 140 ml of methanol, 3.0 ml of concentrated sulfuric acid were added. The mixture was heated at reflux temperature for 1.5 hours, cooled to room temperature and poured into 200 ml of cracked ice with stirring. The formed precipitate was filtered and washed with water to give 2.7g (91.0%) of the desired refined compound.

Melting point: 291°C

IR spectrum ($\nu_{max}^{KBr}$) $cm^{-1}$: 1790, 1745, 1730

NMR spectrum ($DMSO-d_6$) δ ppm:

2.00 - 2.83 (2H, m)
3.83 (3H, s)
5.40 (1H, dd)
6.90 - 7.50 (3H, m)
8.50 (1H, s)
11.77 (1H, broad s)

Mass spectrum (EI/DI) m/z: 294 ($M^+$), 264, 234, 219, 192, 164, 137

Elementary analysis: $C_{13}H_{11}FN_2O_5$

Cal. : C, 53.06; H, 3.77; N, 9.52
Found: C, 53.07; H, 3.62; N, 9.56

Example 11

6-Fluoro-2,3-dihydro-2',5'-dioxo-spiro[4H-1-benzopyran-4,4'-imidazolidine]-2-carboxylic acid 3,6,9,12-tetraoxatridecyl ester

A mixture consisting of 6-fluoro-2,3-dihydro-2',5'-dioxo-spiro[4H-1-benzopyran-4,4'-imidazolidine]-2-carboxylic acid (2.8g, 0.01 mol) (obtained through the process as described in Example 1), 3,6,9,12-tetraoxatridecanol (2.1g, 0.01 mol) and p-toluenesulfonic acid (1.9g, 0.01 mol) in 80 ml of toluene was heated at reflux temperature for 7.0 hours under a Dean-Stark trap. The reaction mixture was evaporated in vacuo to give a semi-solid residue which was partitioned between 50 ml of chloroform and 50 ml of water. The organic layer was dried over anhydrous sodium sulfate, filtered and evaporated in vacuo to give a pale yellow oil which was chromatographed on silica gel, eluting with ethyl acetate to give 3.9g (82.5%) of the desired refined compound as a colorless oil.

IR spectrum ($\nu_{max}^{neat}$) $cm^{-1}$: 3250, 3070, 2880, 1780, 1720

NMR spectrum ($CDCl_3$) δ ppm:

2.47 - 2.83 (2H, m)
3.37 (3H, s)
3.57 - 4.00 (14H, m)
4.33 - 4.60 (2H, m)
5.45 (1H, dd)
6.83 - 7.30 (3H, m)

Mass spectrum (EI/DI) m/z: 470 ($M^+$), 306

Example 12

6-Fluoro-2,3-dihydro-2-hydroxymethyl-spiro[4H-1-benzopyran-4,4'-imidazolidine]-2',5'-dione

To a solution of lithium aluminum hydride (2.3g, 0.06 mol) in 100 ml of tetrahydrofuran, a solution of 6-fluoro-2,3-dihydro-2',5'-dioxo-spiro[4H-1-benzopyran-4,4'-imidazolidine)-2-carboxylic acid methyl ester (11.7g, 0.04 mol)(obtained through the process as described in Example 10) in 100 ml of tetrahydrofuran was added at 5° C. After stirring for 20 hours at room temperature (15 to 20° C), the reaction mixture was poured into 300 ml of cracked ice with stirring. The aqueous solution was acidified to pH I under cooling (10 to 15° C) by addition of concentrated hydrochloric acid and extracted with 400 ml of ethyl acetate. The organic layer was washed with water, dried over anhydrous sodium sulfate, filtered and evaporated in vacuo to give a solid which was recrystallized from methanol to give 8.7g (82.0%) of the desired refined compound.

Melting point: 224 - 225°C

IR spectrum ($\nu_{max}^{KBr}$) $cm^{-1}$: 3360, 1760, 1720

Mass spectrum (EI/DI) m/z: 266 ($M^+$), 248, 219, 205, 192, 164, 137

Elementary analysis: $C_{12}H_{11}FN_2O_4$

Cal. : C, 54.14; H, 4.16; N, 10.52

Found: C, 53.98; H, 4.34; N, 10.35

Example 13

2-Chloromethyl-6-fluoro-2,3-dihydro-spiro[4H-1-benzopyran-4,4'-imidazolidine]-2',5'-dione

To a solution of 6-fluoro-2,3-dihydro-2-hydroxymethyl-spiro[4H-1-benzopyran-4,4'-imidazolidine]-2',5'-dione (2.66g, 0.01 mol)(obtained through the process as described in Example 12) in 20 ml of N,N-dimethylformamide, 1.19g (0.01 mol) of thionylchloride were added, stirred the mixture for 2.0 hours at 20° C and further stirred for 4 hours at 80 to 85° C. After cooling, the reaction mixture was poured into 100 ml of cracked ice and a forming precipitate was filtered. The precipitate was partitioned between 70 ml of ethyl acetate and 50 ml of water. The organic layer was dried over anhydrous sodium sulfate, filtered and evaporated in vacuo to give a yellowish solid which was chromatographed on silica gel, eluting with ethyl acetate and n-hexane (2 : 1) to give 2.42g (85.1%) of the desired refined compound.

Melting point: 212 - 214°C

NMR spectrum (DMSO-$d_6$) δ ppm:

| | |
|---|---|
| 2.33 | (2H, m) |
| 4.07 | (2H, m) |
| 5.07 | (1H, m) |
| 6.93 - 7.47 | (3H, m) |
| 8.53 | (1H, broad s) |
| 11.07 | (1H, broad s) |

Mass spectrum (EI/DI) m/z: 284 ($M^+$), 248, 219, 205, 177, 164, 137

Elementary analysis: $C_{12}H_{10}ClFN_2O_3$

Cal. : C, 50.63; H, 3.54; N, 9.84

Found: C, 50.77; H, 3.40; N, 9.71

Example 14

6-Fluoro-2-fluoromethyl-2,3-dihydro-spiro[4H-1-benzopyran-4,4'-imidazolidine]-2',5'-dione

To a solution of diethylaminosulfur trifluoride (4.09g, 0.025 mol) in 15 ml of anhydrous tetrahydrofuran, a solution of 6-fluoro-2,3-dihydro-2-hydroxymethyl-spiro[4H-1-benzopyran-4,4'-imidazolidine]-2',5'-dione (3.99g, 0.015 mol)(obtained through the process as described in said Example 12) in 80 ml of anhydrous tetrahydrofuran was added at temperature below -50 °C. The mixture was then warmed to room temperature and stirred for 4.5 hours at room temperature. The solvent was evaporated in vacuo and the remaining residue was partitioned between 40 ml of water and 40 ml of ethyl acetate. The organic layer was washed with water, dried over anhydrous sodium sulfate, and evaporated in vacuo to dryness. The remaining residue was chromatographed on silica gel, eluting with ethyl acetate - n-hexane (1 : 1) to give 1.43g (35.5%) of the desired refined compound.

Melting point: 183 - 185℃

IR spectrum ($\nu_{max}^{KBr}$) $cm^{-1}$: 1780, 1730, 1495

NMR spectrum (DMSO-$d_6$) δ ppm:

1.83 - 2.43 (2H, m)

3.90 - 5.47 (3H, m)

6.80 - 7.43 (3H, m)

8.50 (1H, broad s)

11.03 (1H, broad s)

Mass spectrum (EI/DI) m/z: 268 ($M^+$), 248, 219, 205, 197, 192, 177 164, 137

Example 15

2-Bromomethyl-6-fluoro-2,3-dihydro-spiro[4H-1-benzopyran-4,4'-imidazolidine]-2',5'-dione

To a solution of 6-fluoro-2,3-dihydro-2-hydroxymethyl-spiro[4H-1-benzopyran-4,4'-imidazolidine]-2';5'-dione (3.6g, 0.014 mol)(obtained through the process as described in said Example 12) in 28.0 ml of N,N-dimethylformamide, 3.47g (0.017 mol) of thionylbromide were added at temperature below 10 °C. The mixture was stirred for 2.0 hours at 20 °C and further stirred for 1.5 hours at 80 °C. After cooling, the reaction mixture was poured into 40 ml of cracked ice and extracted with ethyl acetate. The organic layer was washed with water, dried over anhydrous sodium sulfate, filtered and evaporated in vacuo to give a solid which was recrystallized from acetone - n-hexane (1 : 2) to give 3.40g (77.3%) of the desired refined compound.

Melting point: 209 - 211℃

IR spectrum ($\nu_{max}^{KBr}$) $cm^{-1}$: 1780, 1740, 1495

NMR spectrum (DMSO-$d_6$) δ ppm:

1.87 - 2.43 (2H, m)
3.73 - 4.03 (2H, m)
4.73 - 5.20 (1H, m)
6.83 - 7.47 (3H, m)
8.53 (1H, broad s)
11.05 (1H, broad s)

Mass spectrum (EI/DI) m/z: 328 ($M^+$), 248, 219, 206, 178, 164, 137

Reference Example 2

2-Adidomethyl-6-fluoro-2,3-dihydro-spiro[4H-1-benzopyran-4,4'-imidazolidine]-2',5'-dione

A mixture consisting of 2-chloromethyl-6-fluoro-2,3-dihydro-spiro[4H-1-benzopyran-4-4'-imidazolidine]-2',5'-dione (4.26g, 0.015 mol) (obtained through the process as described in said Example 13), and sodium iodide (600mg, 0.004 mol), sodium iodide (1.63g, 0.023 mol) in 20 ml of N,N-dimethylformamide was heated at reflux temperature for 1.5 hours and then the reaction mixture was poured into 50 ml of cracked ice. The resulting precipitate was filtered and dissolved in ethyl acetate to obtain an extract. The extract was washed with water, dried over anhydrous sodium sulfate, filtered and evaporated in vacuo to give a solid which was chromatographed on silica gel, eluting with ethyl acetate to give 3.06g (70.1%) of the desired refined compound.

NMR spectrum (DMSO-$d_6$) δ ppm:

2.00 - 2.40 (2H, dd)
3.56 - 3.93 (2H, m)
4.83 - 5.26 (1H, m)
6.86 - 7.50 (3H, m)
8.48 (1H, s)
11.07 (1H, broad s)

Mass spectrum (EI/DI) m/z: 291 ($M^+$), 248, 192

Example 16

2-Aminomethyl-6-fluoro-2,3-dihydro-spiro[4H-1-benzopyran-4,4'-imidazolidine]-2',5'-dione

To a solution of 20% Pd-C (0.6g, 5.6 mmol) in 20 ml of 50% aqueous ethanol, a solution of 2-adidomethyl-6-fluoro-2,3-dihydro-spiro[4H-1-benzopyran-4,4'-imidazolidine]-2',5'-dione (3.0g, 0.01 mol)-(obtained through the process as described in Reference Example 2) in 160 ml of ethanol was added at room temperature. The mixture was hydrogenated for 16 hours at atmospheric pressure. After filtration, the filtrate was evaporated in vacuo to give a solid which was recrystallized from ethanol to give 2.5g (84.0%) of the desired refined compound.

Melting point: 231 - 233°C

IR spectrum ($\nu_{max}^{KBr}$) $cm^{-1}$: 1775, 1725

NMR spectrum ($DMSO-d_6$) $\delta$ ppm:

1.90 - 2.30 (2H, m)

2.82 (2H, d)

4.33 - 5.00 (1H, m)

4.83 - 6.00 (1H, broad)

6.77 - 7.43 (3H, m)

Mass spectrum (EI/DI) m/z: 265 ($M^+$), 248

Elementary analysis: $C_{12}H_{12}FN_3O_3$

Cal. : C, 54.34; H, 4.56; N, 15.84

Found: C, 54.03; H, 4.52; N, 15.45

Reference Example 3

4-Chloro-6-fluoro-3,4-dihydro-2H-1-benzopyran-4-carboxamide

A mixture consisting of 6-fluoro-4-hydroxy-3,4-dihydro-2H-1-benzopyran-4-carboxamide (60mg, 0.28 mmol) and thionylchloride (60mg, 0.28 mmol) in 1.0 ml of diethyl ether was stirred for 5.0 hours at 28° C. The reaction mixture was evaporated in vacuo to give a pale yellowish oil which was chromatographed on silica gel, eluting with ethyl ether to give 43mg (67.2%) of the desired refined compound.

Melting point: 93 - 95°C

IR spectrum ($\nu_{max}^{KBr}$) $cm^{-1}$: 3480, 3420, 1680

NMR spectrum ($CDCl_3$) $\delta$ ppm:

2.10 - 2.57 (1H, m)

2.73 - 3.33 (1H, m)

4.39 (2H, m)

6.50 - 7.27 (5H, m)

Mass spectrum (EI/DI) m/z: 229 ($M^+$), 193, 185

Example 17

6-Fluoro-2,3-dihydro-2'-thioxo-spiro(4H-1-benzopyran-4,4'-imidazolidine)-5'-one

A mixture consiting of 4-chloro-6-fluoro-3,4-dihydro-2H-1-benzopyran-4-carboxamide (10.0g, 0.044 mol)-(obtained through the process described in Reference Example 3) and potassium thiocyanate (13.4g) in 150

ml of acetic acid was heated at reflux temperature for 2.5 hours. The reaction mixture was evaporated in vacuo to dryness and the remaining residue was partitioned between 100 ml of ethyl acetate and 100 ml of water. The organic layer was dried over anhydrous sodium sulfate, filtered and evaporated in vacuo to dryness. The remaining residue was chromatographed on silica gel, eluting with ethyl ether - n-hexane (1 : 1) to give 9.3g (83.7%) of the desired refined compound.

Melting point: 149 - 153°C

IR spectrum ($\nu_{max}^{KBr}$) $cm^{-1}$: 3450, 1745

NMR spectrum ($CDCl_3$) $\delta$ ppm:

2.13 - 2.52 (2H, m)
4.10 - 4.83 (2H, m)
6.56 - 7.17 (3H, m)
8.33 (1H, broad s)
9.60 (1H, broad s)

Mass spectrum (EI/DI) m/z: 252 ($M^+$), 193, 165

Pharmaceutical Agent Preparation Example 1

A dry solid pharmaceutical composition was prepared by blending the following materials together.

| | |
|---|---|
| Product of Example 1 | 50 (parts by weight) |
| Sodium citrate | 25 |
| Alginic acid | 10 |
| Polyvinylpyrrolidone | 10 |
| Magnesium stearate | 5 |

After throughly blended the dry composition, 1000 tablets were punched from the resulting mixture, each of which had a size so as to contain 50mg of the active ingredient.

Pharmaceutical Agent Preparation Examples 2 to 5

Tablets were prepared by the procedure described in Pharmaceutical Agent Preparation Example 1, except that the product of Examples 2, 3, 5 or 8 was employed as the active ingredient in the place of the product of Example 1.

Pharmalogical Test Example 1

Some spiro-3-heteroazolidine compounds according to the invention were tested to evaluate their ability of reduction or inhibition of aldose reductase enzyme activity, in accordance with the procedure proposed by Kador and Sharpless ["Biophysical Chemistry" 8, 81 (1978)].

Using water-soluble extracts of rat lenses, the inhibitions of these compounds were determined. Results are shown in the following Table 1 in terms of percent inhibition of enzyme activity with respect to various concentrations of $10^{-5}$ - $10^{-8}$M. $IC_{50}$ represents the concentration of inhibitor which gives 50% inhibition.

## Table 1

| Compound | Percent Inhibition (%) | | | | $IC_{50}$ (M) |
|---|---|---|---|---|---|
| | $10^{-8}$M | $10^{-7}$M | $10^{-6}$M | $10^{-5}$M | |
| Product of | | | | | |
| Example 1 | | 9 | 47 | 77 | $6.0 \times 10^{-6}$ |
| 2 | | 15 | 85 | 93 | $4.0 \times 10^{-7}$ |
| 3 | | 26 | 78 | 71 | $5.0 \times 10^{-7}$ |
| 5 | | 10 | 29 | 61 | $5.0 \times 10^{-6}$ |
| 8 | 14 | 65 | 81 | 81 | $7.0 \times 10^{-8}$ |
| 12 | | 13 | 28 | 85 | $4.0 \times 10^{-6}$ |
| 13 | | 3 | 51 | 90 | $1.0 \times 10^{-6}$ |

Pharmacological Test Example 2

Some spiro-3-heteroazolidine compounds according to the invention were tested to evaluate their ability of reduction or inhibition of polyol increase in sciatic nerve of galactosemic rats. To the rats, 30% galactose diet was fed and said compounds were orally administered at a dose of 10 mg/kg once a day for a period of 8 days. Control animals received the galactose diet and were not administered such compound. One day after final administration (on 9th day from the first administration), sciatic nerves were removed for galactitol determination.

Results are shown in following Table 2 in terms of percent inhibition as compared to galactitol increase of the control animals.

## Table 2

| Compound | Percent Inhibition (%) |
|---|---|
| Product of | |
| Example 1 | 36 |
| 2 | 85 |
| 3 | 31 |
| 5 | 70 |
| 8 | 26 |
| 12 | 40 |
| 13 | 88 |

**Claims**

1. A spiro-3-heteroazolidine compound represented by the formula

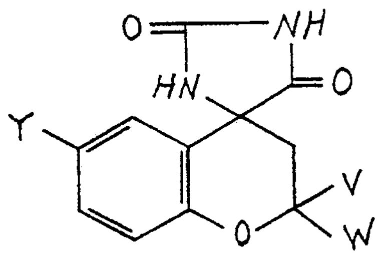

or

(I-A)

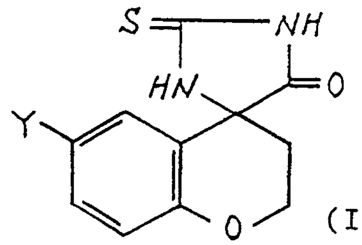

(I-B)

wherein one of V and W represents a hydrogen atom, the other of V and W represents a hydroxymethyl radical, aminomethyl radical, halomethyl radical, -COOR, or

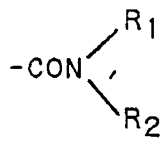

Y represents a hydrogen atom or halogen atom, R is a hydrogen atom, alkyl group having 1 to 6 carbon atoms or $-(CH_2CH_2O)_4)CH_3$, $R_1$ and $R_2$ are the same or different and each represents a hydrogen atom, alkyl radical having 1 to 6 carbon atoms, $-(CH_2CH_2O)_4CH_3$, or methoxy substituted phenyl radical,
and pharmaceutically acceptable salts thereof.

2. A compound as claimed in Claim 1, wherein said compound is 6-fluoro-2,3-dihydro-2'-thioxo-spiro[4H-1-benzopyran-4,4'-imidazolidine]-5'-one.

3. A compound as claimed in Claim 1, wherein said compound is 6-fluoro-2,3-dihydro-2',5'-dioxo-spiro[4H-1-benzopyran-4,4'-imidazolidine]-2-carboxylic acid.

4. A compound as claimed in Claim 1, wherein said compound is 6-fluoro-2,3-dihydro-2',5'-dioxo-spiro[4H-1-benzopyran-4,4'-imidazolidine]-2-carboxylic acid methyl ester.

5. A compound as claimed in Claim 1, wherein said compound is 6-fluoro-2,3-dihydro-2',5'-dioxo-spiro[4H-1-benzopyran-4,4'-imidazolidine]-2-carboxylic acid 3,6,9,12-tetraoxatridecyl ester.

6. A compound as claimed in Claim 1, wherein said compound is 6-fluoro-2,3-dihydro-2',5'-dioxo-spiro[4H-1-benzopyran-4,4'-imidazolidine]-2-carboxamide.

7. A compound as claimed in Claim 1, wherein said compound is 6-fluoro-2,3-dihydro-N-methyl-2',5'-dioxo-spiro[4H-1-benzopyran-4,4'-imidazolidine]-2-carboxamide.

8. A compound as claimed in Claim 1, wherein said compound is 6-fluoro-2,3-dihydro-N,N-dimethyl-2',5'dioxo-spiro[4H-1-benzopyran-4,4'-imidazolidine]-2-carboxamide.

9. A compound as claimed in Claim 1, wherein said compound is 6-fluoro-2,3-dihydro-N-ethyl-2',5'-dioxo-spiro[4H-1-benzopyran-4,4'-imidazolidine]-2-carboxamide.

10. A compound as claimed in Claim 1, wherein said compound is 6-fluoro-2,3-dihydro-N-propyl-2',5'-dioxo-spiro[4H-1-benzopyran-4,4'-imidazolidine]-2-carboxamide.

11. A compound as claimed in Claim 1, wherein said compound is 6-fluoro-2,3-dihydro-N-butyl-2',5'-dioxo-spiro[4H-1-benzopyran-4,4'-imidazolidine]-2-carboxamide.

**12.** A compound as claimed in Claim 1, wherein said compound is 6-fluoro-2,3-dihydro-N-(4-methoxyphenyl)-2',5'-dioxo-spiro[4H-1-benzopyran-4,4'-imidazolidine]-2-carboxamide.

**13.** A compound as claimed in Claim 1, wherein said compound is 6-fluoro-2,3-dihydro-N-(3,6,9,12-tetraoxatridecyl)-2',5'-dioxo-spiro[4H-1-benzopyran-4,4'-imidazolidine]-2-carboxamide.

**14.** A compound as claimed in Claim 1, wherein said compound is 6-fluoro-2,3-dihydro-2-hydroxymethyl-spiro[4H-1-benzopyran-4,4'-imidazolidine]-2',5'-dione.

**15.** A compound as claimed in Claim 1, wherein said compound is 2-chloromethyl-6-fluoro-2,3-dihydro-spiro[4H-1-benzopyran-4,4'-imidazolidine]-2',5'-dione.

**16.** A compound as claimed in Claim 1, wherein said compound is 2-fluoromethyl-6-fluoro-2,3-dihydro-spiro[4H-1-benzopyran-4,4'-imidazolidine]-2',5'-dione.

**17.** A compound as claimed in Claim 1, wherein said compound is 2-bromomethyl-6-fluoro-2,3-dihydro-spiro[4H-1-benzopyran-4,4'-imidazolidine]-2',5'-dione.

**18.** A compound as claimed in Claim 1, wherein said compound is 2-aminomethyl-6-fluoro-2,3-dihydro-spiro[4H-1-benzopyran-4,4'-imidazolidine]-2',5'-dione.

**19.** A process of preparing a spiro-3-heteroazolidine compound represented by the formula

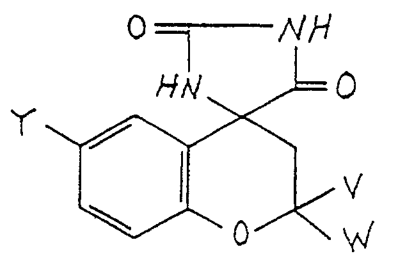

(I-A)

wherein V, W and Y are as defined in Claim 1,
which comprises reacting a compound represented by the formula

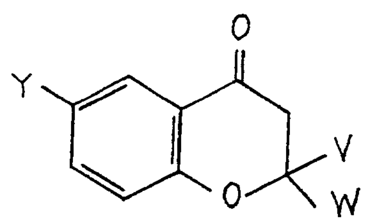

(II)

wherein V, W and Y are as defined in Claim 1,
with a metallic cyanide and ammonium carbonate and, if necessary, converting the resulting reaction product into the salt.

**20.** A process of preparing a spiro-3-heteroazolidine compound represented by the formula

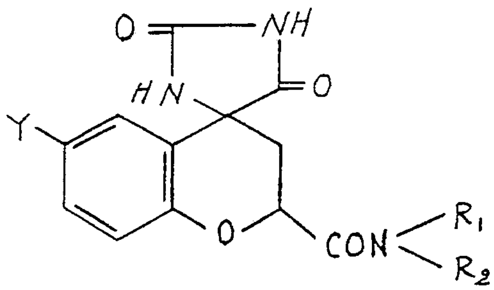

(I-A-2)

wherein Y, $R_1$ and $R_2$ are as defined in Claim 1,
which comprises reacting a compound represented by the formula

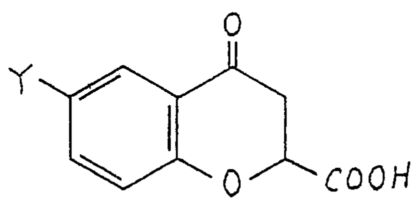

wherein Y is as defined in Claim 1,
with a metallic cyanide and ammonium carbonate, reacting the resulting reaction product represented by the formula

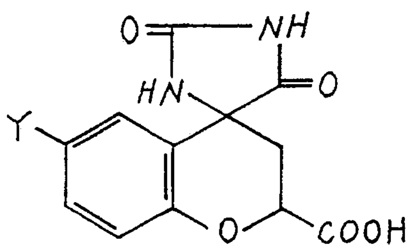

(I-A-1)

wherein Y is as defined in Claim 1,
with a compound represented by the formula

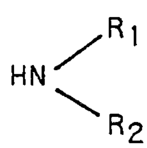

wherein $R_1$ and $R_2$ are as defined in Claim 1,
in the presence of a condensing agent and, if necessary, converting the resulting reaction product into the salt.

**21.** A process of preparing a spiro-3-heteroazolidine compound represented by the formula

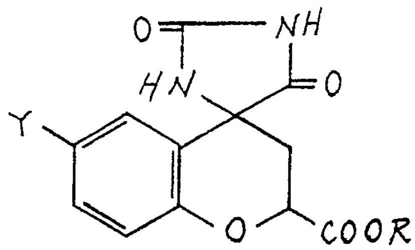

(I-A-3)

wherein Y and R are as defined in Claim 1,
which comprises reacting a compound represented by the formula

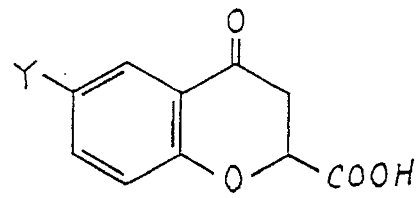

wherein Y is as defined in Claim 1,
with a metallic cyanide and ammonium carbonate, reacting the resulting reaction product represented by the formula

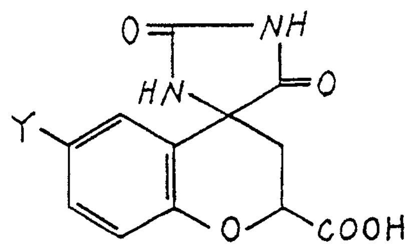

(I-A-1)

wherein Y is as defined in Claim 1,
with a compound represented by the formula

ROH

wherein R is as defined in Claim 1,
in the presence of a condensing agent and, if necessary, converting the resulting reaction product into the salt.

22. A process of preparing a spiro-3-heteroazolidine compound represented by the formula

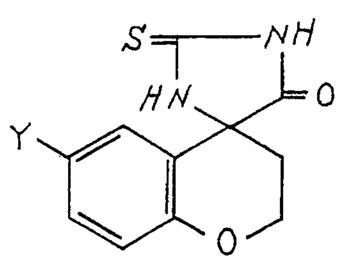

wherein Y is as defined in Claim 1,

which comprises reacting a compound represented by the formula

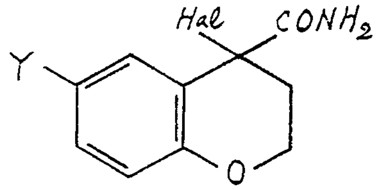

wherein Y is as defined in Claim 1 and Hal is a halogen atom, with potassium thiocyanate and, if necessary, converting the resulting reaction product into the salt.

23. A medicine for preventing or curing complications caused by diabetes, which comprises in effective amount at least one compound as claimed in any of Claims 1 to 18.

**Revendications**

1. Composé spiro-3-hétéroazolidine représenté par la formule

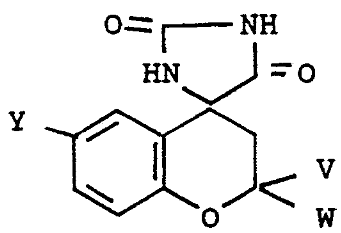

ou

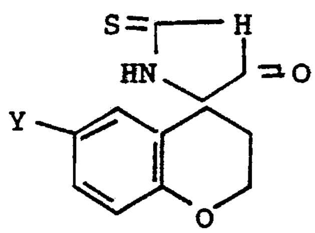

(I-A) (I-B)

où un des groupes V et W représente un atome d'hydrogène, l'autre des groupes V et W représente un radical hydroxyméthyle, un radical aminométhyle, un radical halométhyle,

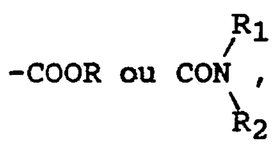

Y représente un atome hydrogène ou un atome halogène, R est un atome d'hydrogène, un groupe alkyle ayant de 1 à 6 atomes de carbone ou $-(CH_2CH_2O)_4)CH_3$, $R_1$ et $R_2$ sont les mêmes ou différents et chacun représente un atome d'hydrogène, un radical alkyle ayant de 1 à 6 atomes de carbone, $-(CH_2CH_2O)_4CH_3$ ou un radical phényle avec un substituant méthoxy, et les sels de ceux-ci, acceptables sur le plan pharmaceutique.

2. Composé selon la revendication 1, où ledit composé est la 6-fluoro-2,3-dihydro-2'-thioxo-spiro[4H-1-benzopyran-4,4'-imidazolidine]-5'-one.

3. Composé selon la revendication 1, où ledit composé est l'acide 6-fluoro-2,3-dihydro-2',5'-dioxo-spiro-[4H-1-benzopyran-4,4'-imidazolidine]-2 carboxylique.

4. Composé selon la revendication 1, où ledit composé est l'ester méthylique de l'acide 6-fluoro-2,3-dihydro-2',5'-dioxo-spiro [4H-1-benzopyran-4,4'-imidazolidine]-2-carboxylique.

5. Composé selon la revendication 1, où ledit composé est l'ester 3,6,9,12-tétraoxatridécylique de l'acide 6-fluoro-2,3-dihydro-2',5'-dioxo-spiro[4H-1-benzopyran-4,4' -imidazolidine]-2-carboxylique.

6. Composé selon la revendication 1, où ledit composé est la 6-fluoro-2,3-dihydro-2',5'-dioxo-spiro[4H-1-benzopyran-4,4'-imidazolidine]-2-carboxamide.

7. Composé selon la revendication 1, où ledit composé est la 6-fluoro-2,3-dihydro-N-méthyl-2',5'-dioxo-spiro[4H-1-benzopyran-4,4'-imidazolidine]-2-carboxamide.

8. Composé selon la revendication 1, où ledit composé est la 6-fluoro-2,3-dihydro-N,N-diméthyl-2',5'-dioxo-spiro[4H-1-benzopyran-4,4'-imidazolidine]-2-carboxamide.

9. Composé selon la revendication 1, où ledit composé est la 6-fluoro-2,3-dihydro-N-éthyl-2',5'-dioxo-spiro[4H-1-benzopyran-4,4'-imidazolidine]-2-carboxamide.

10. Composé selon la revendication 1, où ledit composé est la 6-fluoro-2,3-dihydro-N-propyl-2',5'-dioxo-spiro[4H-1-benzopyran-4,4'-imidazolidine]-2-carboxamide.

11. Composé selon la revendication 1, où ledit composé est la 6-fluoro-2,3-dihydro-N-butyl-2',5'-dioxo-spiro[4H-1-benzopyran-4,4'-imidazolidine]-2-carboxamide.

12. Composé selon la revendication 1, où ledit composé est la 6-fluoro-2,3-dihydro-N-(4-méthoxyphényl)-2',5'-dioxo-spiro[4H-1-benzopyran-4,4'-imidazolidine]-2-carboxamide.

13. Composé selon la revendication 1, où ledit composé est la 6-fluoro-2,3-dihydro-N-(3,6,9,12-tétraoxatridécyl)-2',5'-dioxo-spiro[4H-1-benzopyran-4,4'-imidazolidine]-2-carboxamide.

14. Composé selon la revendication 1, où ledit composé est la 6-fluoro-2,3-dihydro-2-hydroxyméthyl-spiro-[4H-1-benzopyran-4,4'-imidazolidine]-2',5'-dione.

15. Composé selon la revendication 1, où ledit composé est la 2-chlorométhyl-6-fluoro-2,3-dihydro-spiro-[4H-1-benzopyran-4,4'-imidazolidine]-2',5'-dione.

16. Composé selon la revendication 1, où ledit composé est la 2-fluorométhyl-6-fluoro-2,3-dihydro-spiro[4H-1-benzopyran-4,4'-imidazolidine]-2',5'-dione.

17. Composé selon la revendication 1, où ledit composé est la 2-bromométhyl-6-fluoro-2,3-dihydro-spiro-[4H-1-benzopyran-4,4'-imidazolidine]-2',5'-dione.

18. Composé selon la revendication 1, où ledit composé est la 2-aminométhyl-6-fluoro-2,3-dihydro-spiro-[4H-1-benzopyran-4,4'-imidazolidine]-2',5'-dione.

19. Procédé de préparation d'un composé spiro-3-hétéroazolidine représenté par la formule

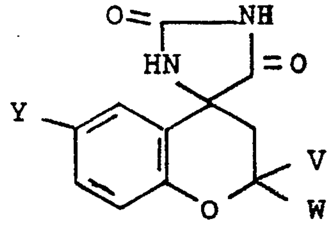

(I - A)

où V, W et Y sont définis comme dans la revendication 1,
qui comprend de faire réagir un composé représenté par la formule

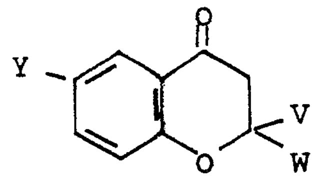

(II)

où V, W et Y sont comme défini dans la revendication 1, avec un cyanure de métal et avec du carbonate d'ammonium et, lorsque cela est nécessaire, de convertir le produit de réaction obtenu en sel.

**20.** Procédé de préparation d'un composé spiro-3-hétéroazolidine représenté par la formule

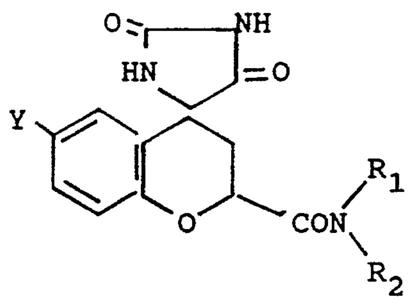

(I - A -2)

où Y, $R_1$ et $R_2$ sont définis comme dans la revendication 1, qui comprend de faire réagir un composé représenté par la formule

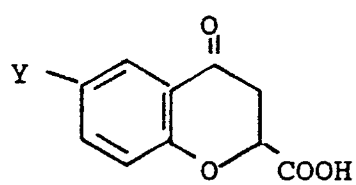

où Y est défini comme dans la revendication 1, avec un cyanure de métal et du carbonate d'ammonium, et à faire réagir le composé obtenu représenté par la formule

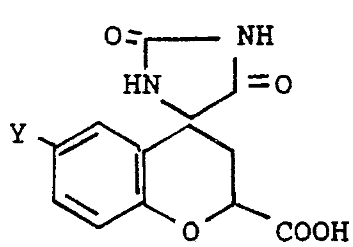

(I-A-1)

où Y est défini comme dans la revendication 1, avec un composé représenté par la formule

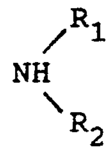

où $R_1$ et $R_2$ sont définis comme dans la revendication 1, en présence d'un agent de condensation et, lorsque cela est nécessaire, de convertir le produit de cette réaction en sel.

**21.** Procédé de préparation d'un composé spiro-3-hétéroazolidine représenté par la formule

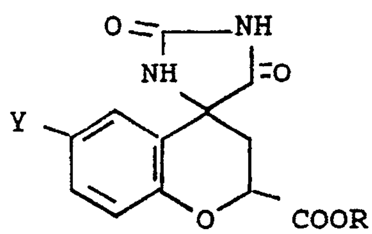

(I-A-3)

où Y et R sont définis comme dans la revendication 1, qui comprend de faire réagir un composé représenté par la formule

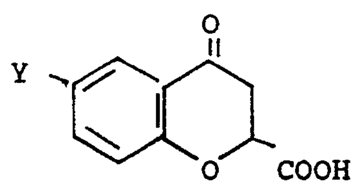

où Y est défini comme dans la revendication 1, avec un cyanure de métal et du carbonate d'ammonium, et de faire réagir le produit de cette réaction représenté par la formule

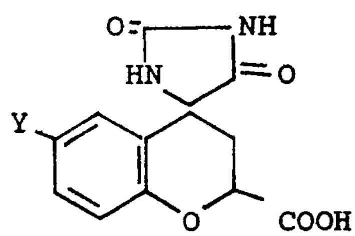

(I-A-1)

où Y est défini comme dans la revendication 1, avec un composé représenté par la formule

ROH

où R est défini comme dans la revendication 1, en présence d'un agent de condensation et, lorsque cela est nécessaire, de convertir le produit résulat de cette réaction en sel.

**22.** Procédé de préparation d'un composé spiro-3-hétéroazolidine représenté par la formule :

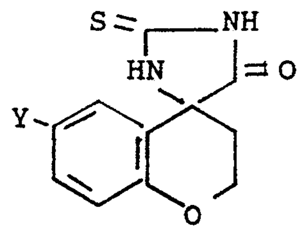

où Y est défini comme dans la revendication 1,
qui comprend de faire réagir un composé représenté par la formule

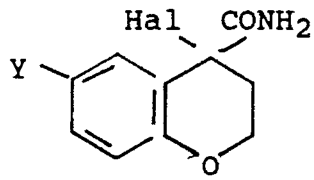

où Y est défini comme dans la revendication 1 et Hal est un atome d'halogène,
avec du thiocyanate de potassium et, lorsque cela est nécessaire, de convertir le produit résultant de cette réaction en sel.

**23.** Médicament pour empêcher ou guérir les complications provoquées par le diabète, qui contient une quantité efficace d'au moins un des composés de l'une quelconque des revendications 1 à 18.

**Ansprüche**

**1.** Eine Spiro-3-Heteroazolidin-Verbindung, dargestellt durch die Formel

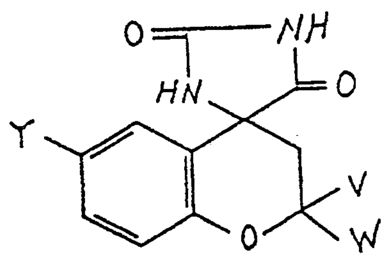

(I-A)

oder

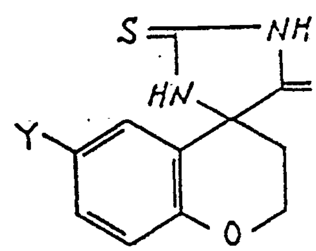

(I-B)

worin eines der V und W ein Wasserstoffatom darstellt, und das andere der V und W ein Hydroxymethyl-Radikal, Aminomethyl-Radikal, Halomethyl-Radikal, -COOR, oder

$R_1$

**- CON** ,

$R_2$

Y ein Wasserstoff- oder Halogenatom, R ein Wasserstoffatom, eine Alkylgruppe mit 1 bis 6 Kohlenstoffatomen oder $-(CH_2CH_2O)_4)CH_3$ darstellt, $R_1$ und $R_2$ sind gleich oder unterschiedlich und jedes stellt ein Wasserstoffatom, Alkylradikal dar mit 1 bis 6 Kohlenstoffatomen, $-(CH_2CH_2O)_4)CH_3$ oder methoxysubstituiertes Phenylradikal,
und deren pharmazeutisch akzeptierbaren Salze.

2. Verbindung nach Patentanspruch 1, worin die Verbindung 6-Fluor-2,3-Dihydro-2'-Thioxo-Spiro[4H-1-Benzopyran-4,4'-Imidazolidin]-5'-eins ist.

3. Verbindung nach Patentanspruch 1, worin die Verbindung 6-Fluor-2,3-Dihydro-2',5'-Dioxo-Spiro[4H-1-Benzopyran-4,4'-Imidazolidin]-2-Karboxylsäure ist.

4. Verbindung nach Patentanspruch 1, worin die Verbindung 6-Fluor-2,3-Dihydro-2',5'-Dioxo-Spiro[4H-1-Benzopyran-4,4'-Imidazolidin]-2-Karbolxylsäure-Methylester ist.

5. Verbindung nach Patentanspruch 1, worin die Verbindung 6-Fluor-2,3-Dihydro-2',5'-Dioxo-Spiro[4H-1-Benzopyran-4,4'-Imidazolidin]-2-Karboxylsäure-3,6,9,12-Tetroaoxatridecylester ist.

6. Verbindung nach patentanspruch 1, worin die Verbindung 6-Fluor-2,3-Dihydro-2',5'-Dioxo-Spiro[4H-1-Benzopyran-4,4'-Imidazolidin]-2-Karboxamid ist.

7. Verbindung nach Patentanspruch 1, worin die Verbindung 6-Fluor-2,3-Dihydro-N-Methyl-2',5'-Dioxo-Spiro[4H-1-Benzopyran-4,4'-Imidazolidin]-2-Karboxamid ist.

8. Verbindung nach Patentanspruch 1, worin die Verbindung 6-Fluor-2,3-Dihydro-N,N-Dimethyl-2',5'-Dioxo-Spiro [4H-1-Benzopyran-4,4'-Imidazolidin]-2-Karboxamid ist.

9. Verbindung nach Patentanspruch 1, worin die Verbindung 6-Fluor-2,3-Dihydro-N-Aethyl-2',5'-Dioxo-Spiro[4H-1-Benzopyran-4,4'-Imidazolidin]-2-Karboxamid ist.

10. Verbindung nach Patentanspruch 1, worin die Verbindung 6-Fluor-2,3-Dihydro-N-Propyl-2',5'-Dioxo-Spiro[4H-1-Benzopyran-4,4'-Imidazolidin]-2-Karboxamid ist.

11. Verbindung nach Patentanspruch 1, worin die Verbindung 6-Fluor-2,3-Dihydro-N-Butyl-2',5'-Dioxo-Spiro[4H-1-Benzopyran-4,4'-Imidazolidin]-2-Karboxamid ist.

12. Verbindung nach Patentanspruch 1, worin die Verbindung 6-Fluor-2,3-Dihydro-N-(4-Methoxyphenyl)-2',5'-Dioxo-Spiro[4H-1-Benzopyran-4,4'-Imidazolidin]-2-Karboxamid ist.

13. Verbindung nach Patentanspruch 1, worin die Verbindung6-Fluor-2,3-Dihydro-N-(3,6,9,12-Tetraoxatridecyl)-2',5'-Dioxo-Spiro[4H-1-Benzopyran-4,4'-Imidazolidin]-2-Karboxamid ist.

14. Verbindung nach Patentanspruch 1, worin die Verbindung 6-Fluor-2,3-Dihydro-2-Hydroxymethyl-Spiro-[4H-1-Benzopyran-4,4'-Imidazolidin]-2',5'-Dion ist.

15. Verbindung nach Patentanspruch 1, worin die Verbindung 2-Chlormethyl-6-Fluor-2,3-Dihydro-Spiro[4H-1-Benzopyran-4,4'-Imidazolidin]-2',5'-Dion ist.

16. Verbindung nach Patentanspruch 1, worin die Verbindung 2-Fluormethyl-6-Fluor-2,3-Dihydro-Spiro[4H-1-Benzopyran-4,4'-Imidazolidin]-2',5'-Dion ist.

17. Verbindung nach Patentanspruch 1, worin die Verbindung Bromomethyl-6-Fluor-2,3-Dihydro-Spiro[4H-1-Benzopyran-4,4'-Imidazolidin]-2',5'-Dion ist.

18. Verbindung nach Patentanspruch 1, worin die Verbindung 2-Aminomethyl-6-Fluor-2,3-Dihydro-Spiro[4H-1-Benzopyran-4,4'-Imidazolidin]-2',5'-Dion ist.

19. Verfahren zur Herstellung einer Spiro-3-Heteroazolidin-Verbindung, dargestellt durch die Formel

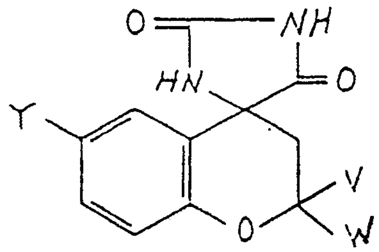

(I-A)

worin V,W und Y so definiert sind, wie im Patentanspruch 1 angegeben, das besteht aus Reagieren einer Verbindung, dargestellt durch die Formel

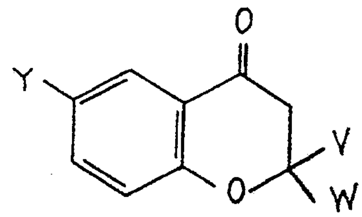

(II)

mit einem metallischen Zyanid und Ammoniumkarbonat und, falls erforderlich, Umwandeln des sich ergebenden Reaktionsproduktes in das Salz.

**20.** Verfahren zur Herstellung einer Spiro-3-Heteroazolidin-Verbindung, dargestellt durch die Formel

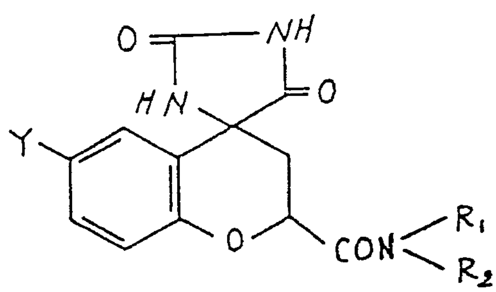

(I-A-2)

worin Y, $R_1$ und $R_2$ so definiert sind, wie im Patentanspruch 1 angegeben, das besteht aus Reagieren einer Verbindung, dargestellt durch die Formel

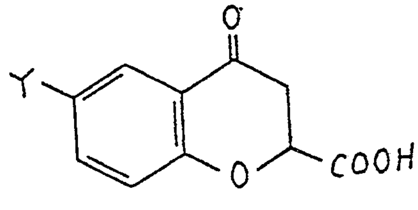

(Z1)

worin Y so definiert sind, wie im Patentanspruch 1 angegeben, mit einem metallischen Zyanid und Ammoniumkarbonat, wobei das sich ergebende Reaktionsprodukt, dargestellt durch die Formel

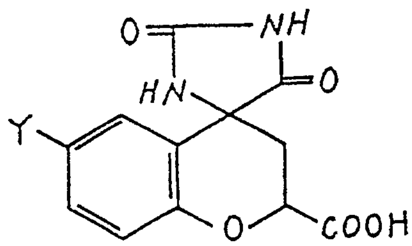

(I-A-1)

in der Y so definiert sind, wie im Patentanspruch 1 angegeben, reagiert mit einer Verbindung, dargestellt durch die Formel

$R_1$

HN

$R_2$

worin $R_1$ und $R_2$ so definiert sind, wie im Patentanspruch 1 angegeben, in Gegenwart eines Kondensationsmittels, und, falls erforderlich, Umwandlung des sich ergebenden Reaktionsproduktes in das Salz.

**21.** Verfahren zur Herstellung einer Spiro-3-Heteroazolidin-Verbindung, dargestellt durch die Formel

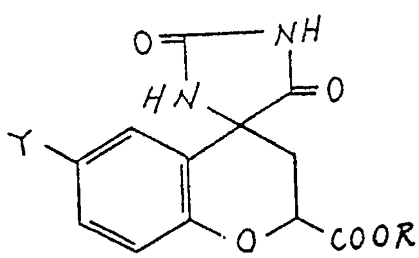

(I-A-3)

worin Y und R so definiert sind, wie im Patentanspruch 1 angegeben, das besteht aus Reagieren einer Verbindung, dargestellt durch die Formel

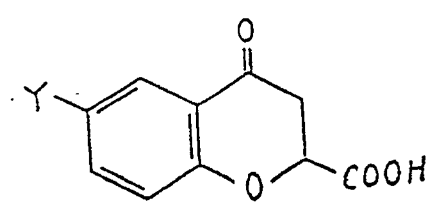

(Z1)

worin Y so definiert ist, wie im Patentanspruch 1 angegeben, mit einem metallischen Zyanid und Ammoniumkarbonat, wobei das sich ergebende Reaktionsprodukt, dargestellt durch die Formel

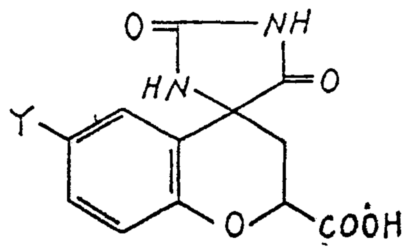

(I-A-1)

worin Y so definiert ist, wie im Patentanspruch 1 angegeben, reagiert mit einer Verbindung, dargestellt durch die Formel

ROH

worin R so definiert ist, wie im Patentanspruch 1 angegeben, in Gegenwart eines Kondensationsmittels, und, falls erforderlich, Umwandeln des sich ergebenden Reaktionsproduktes in das Salz.

**22.** Verfahren zur Herstellung einer Spiro-3-Heteroazolidin-Verbindung, dargestellt durch die Formel

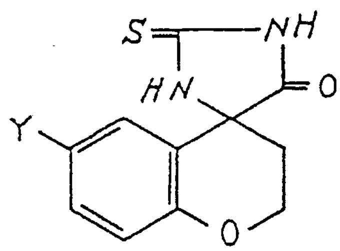

(Z3)

worin Y so definiert ist, wie im Patentanspruch 1 angegeben, das besteht aus Reagieren einer Verbindung, dargestellt durch die Formel

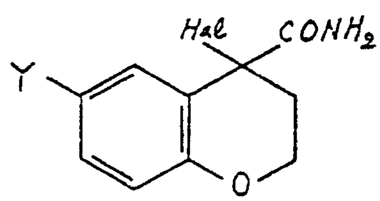

(Z4)

worin Y so definiert ist, wie im Patentanspruch 1 angegeben, und Hal ist ein Halogenatom, mit Kaliumthiozyanat und, falls erforderlich, Umwandeln des sich ergebenden Reaktionsproduktes in das Salz.

**23.** Eine Medizin zur Verhinderung von Heilungskomplikationen verursacht durch Diabetes, die mindestens eine der Verbindungen nach einem der Patentansprüche 1 bis 18 in einer wirksamen Menge enthält.